# EUROPEAN PATENT APPLICATION

(11) **EP 4 451 277 A1**
(43) Date of publication of application: **23.10.2024**
(21) Application number: 24171112.6
(22) Date of filing: 18.04.2024
(51) Int. Cl.: G16B 40/00, G16C 20/30, G16C 20/70, G16H 10/20, G16H 50/20, G16H 70/40

(54) **EVALUATION OF INGREDIENTS FOR TOXICITY WITH MACHINE LEARNING**

(30) Priority: 18.04.2023 US 202363496776 P
(71) Applicant: Sensorygen, Inc., South Pasadena, CA 91030 (US)
(72) Inventor: RAY, Anandasankar, Riverside, CA 92506 (US); KOWALEWSKI, Joel, South Pasadena, CA 91030 (US)
(74) Representative: Gill Jennings & Every LLP

(57) **Abstract**

Systems and methods for evaluating the toxicity of a chemical or chemicals are provided, including evaluating the estimated success or failure in clinical trials and a comprehensive profile of human proteins and biochemical pathways implicated. A user can direct the output toward their intended use case. A web interface and/or an application programming interface can be used, and customization is permitted to build new machine learning models that predict activity on human proteins of interest (e.g., those that are currently not built-in or prepackaged with the application or service).

## Description

### CROSS-REFERENCE TO RELATED APPLICATION

This application claims the benefit of U.S. Provisional Application Serial No. 63/496,776, filed April 18, 2023, the disclosure of which is hereby incorporated by reference in its entirety, including all figures, tables, and drawings.

### BACKGROUND

Supplements and personal care products are not currently subject to significant regulation for short- or long-term impact, either on humans or ecosystems in general. The responsibility therefore lies with the companies producing these products, but animal testing and *in vitro* testing for safety are both costly. Lacking mandatory regulations, such testing is possibly omitted. Government agencies are increasingly studying the ingredients for these products to enhance public awareness; however, these efforts are incapable of addressing all or even most chemicals or ingredients in supplements and personal care products. While databases are available, analysis and visualization tools are sparse or directed at a technical audience, thereby limiting accessibility and the prospect of safety-conscious consumers pressuring companies to sell products with non-toxic ingredients.

Although inferring human toxicity from non-human species is challenging and possibly inaccurate, these data when popularized affect long-term profitability. Two key issues in ingredient and product development and discovery are (1) possibly selling products with genuine safety concerns and (2) possibly selling products with inferred safety concerns. Both may lead to costs that exceed self-imposed safety regulations at early stages of research and development.

### BRIEF SUMMARY

By combining animal testing and machine learning (and/or artificial intelligence), it is possible to create automated pipelines to simulate human toxicity, improving safety where regulation is lacking and/or safeguarding products by avoiding ingredients that may receive future media coverage or appear in studies. Embodiments of the subject invention provide novel and advantageous systems and methods for predicting activity on human proteins important in growth and development, cell proliferation, DNA repair and regulation, as well as reproductive health. Systems and methods can utilize one or more machine learning models that predict potential clinical trial failures for candidate chemicals and/or candidate drugs. Such prediction(s) can be based on expected or actual activity on human proteins. A system can include a processor and a (non-transitory) machine-readable medium (e.g., a (non-transitory) computer-readable medium) in operable communication with the processor and having instructions stored thereon that, when executed, perform steps including running the machine learning model(s) and predicting activity on human proteins and/or predicting potential clinical trial failures for candidate chemicals and/or candidate drugs. The systems and methods of embodiments of the subject invention can be software-based, cloud-based, application (app)-based, and/or web-based.

In an embodiment, a system for predicting clinical trial outcomes for chemicals (e.g., drugs) can comprise a processor and a machine-readable medium in operable communication with the processor and having instructions stored thereon that, when executed by the processor, perform the following steps: utilizing at least one machine learning model on known toxicological test results to develop a prediction model; receiving data of a candidate chemical; and utilizing the prediction model on the candidate chemical to predict a clinical trial outcome for the candidate chemical. The known toxicological test results can be uploaded to the system by, for example, a user of the system. The known toxicological test results can be test results on human protein targets. The system can further comprise a display in operable communication with the processor, and the instructions when executed can further perform the step of displaying the predicted clinical trial outcome on the display. The instructions when executed can further perform the step of generating summary reports. The summary reports can comprise broad toxicological risk categories. The summary reports can comprise pathway analysis and estimating for use biochemical pathways that are affected by the candidate chemical. The summary reports can comprise at least one of estimated vapor pressure, blood brain barrier permeability, and toxicological estimates. The summary reports can comprise additional whole organ or *in vivo* toxicological estimates including at least one of dermal absorption and irritation, cardiac and kidney toxicity, mammalian LD50, a measure of lethality, liver toxicity, and eye irritation. The candidate chemical can be, for example, a drug.

In another embodiment, a method for predicting clinical trial outcomes for chemicals (e.g., drugs) can comprise: utilizing (e.g., by a processor) at least one machine learning model on known toxicological test results to develop a prediction model; receiving (e.g., by the processor) data of a candidate chemical; and utilizing (e.g., by the processor) the prediction model on the candidate chemical to predict a clinical trial outcome for the candidate chemical. The known toxicological test results can be uploaded to the system by, for example, a user of the system. The known toxicological test results can be test results on human protein targets. The method can further comprise displaying (e.g., by the processor) the predicted clinical trial outcome on the display. The method can further comprise generating (e.g., by the processor) summary reports. The summary reports can comprise broad toxicological risk categories. The summary reports can comprise pathway analysis and estimating for use biochemical pathways that are affected by the candidate chemical. The summary reports can comprise at least one of estimated vapor pressure, blood brain barrier permeability, and toxicological estimates. The summary reports can comprise additional whole organ or *in vivo* toxicological estimates including at least one of dermal absorption and irritation, cardiac and kidney toxicity, mammalian LD50, a measure of lethality, liver toxicity, and eye irritation. The candidate chemical can be, for example, a drug.

### BRIEF DESCRIPTION OF DRAWINGS

**Figure 1** is a bar chart of metric value for auc, spec, and sens, depicting training on about 5000 samples to predict clinical status from toxicity.

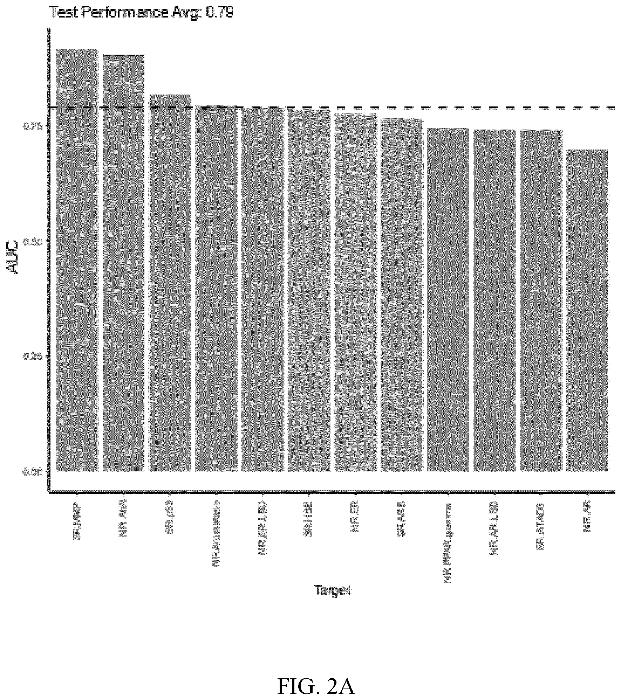

**Figure 2A** is a bar chart showing test performance for auc for different targets. The average test performance was 0.79.

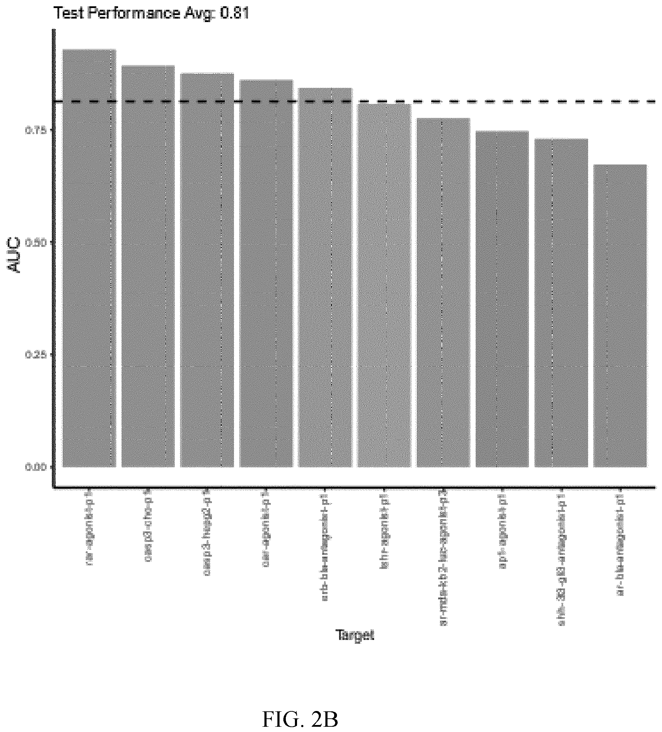

**Figure 2B** is a bar chart showing test performance for auc for different targets. The average test performance was 0.81.
**Figures 3A-3D** combine to form a table of protocols, assay targets (with target categories), and cell lines (with cell types).

### DETAILED DESCRIPTION

Embodiments of the subject invention provide novel and advantageous systems and methods for predicting activity on human proteins important in growth and development, cell proliferation, DNA repair and regulation, as well as reproductive health. Systems and methods can utilize one or more machine learning models that predict potential clinical trial failures for candidate chemicals and/or candidate drugs. Such prediction(s) can be based on expected or actual activity on human proteins. A system can include a processor and a (non-transitory) machine-readable medium (e.g., a (non-transitory) computer-readable medium) in operable communication with the processor and having instructions stored thereon that, when executed, perform steps including running the machine learning model(s) and predicting activity on human proteins and/or predicting potential clinical trial failures for candidate chemicals and/or candidate drugs. The systems and methods of embodiments of the subject invention can be software-based, cloud-based, application (app)-based, and/or web-based.

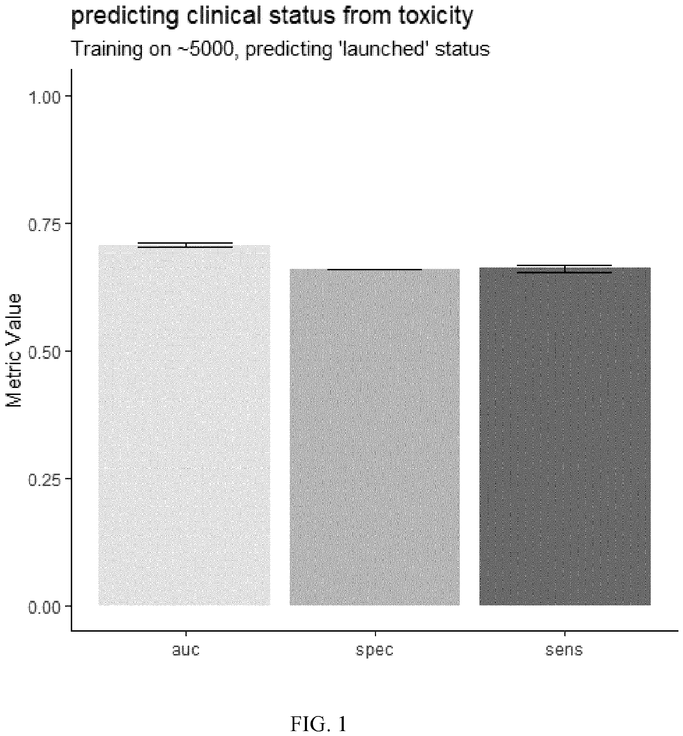

In many embodiments, a user can input a chemical, drug, list of chemicals, list of drugs, or combined list of chemicals and drugs to estimate or predict clinical trial success or failure with high accuracy (e.g., at least 70%, as shown in Figure 1), particularly relative to chance. A user can retrieve toxicological summaries for the uploaded chemical, drug, or list. Reports can be supplied as, for example, one or more interactive dashboard. Such interactive dashboard(s) can be downloaded (e.g., as html file(s)) and used offline.

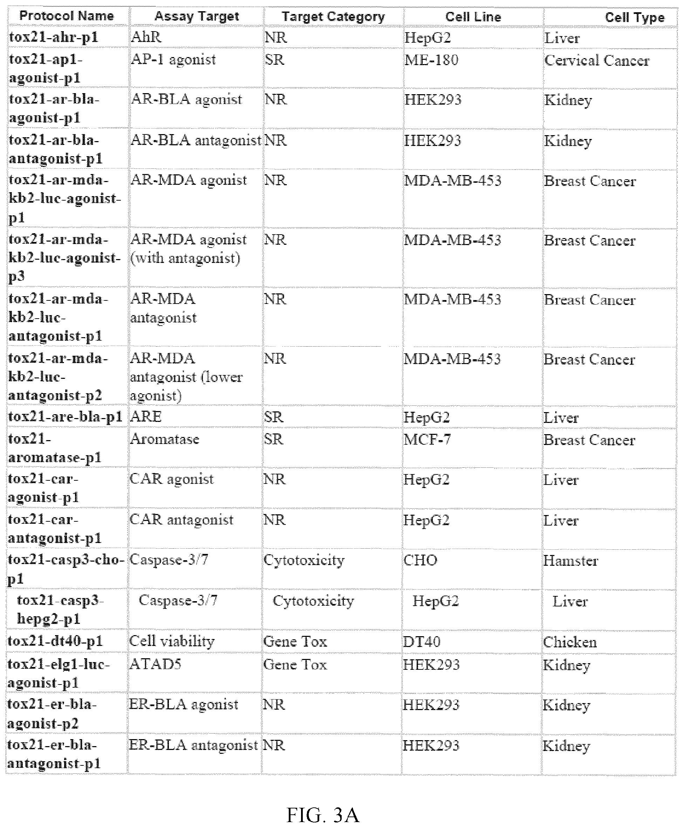

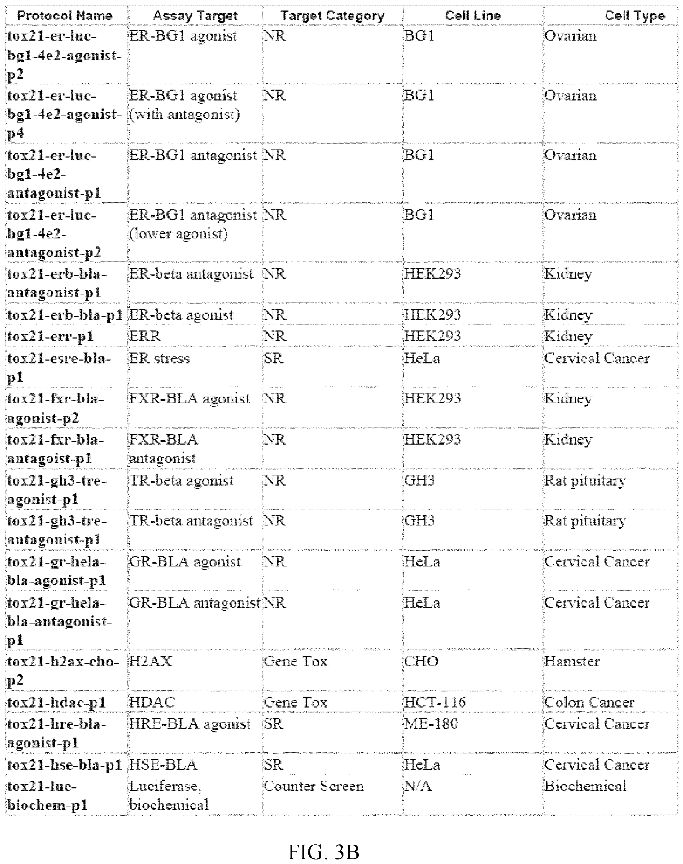

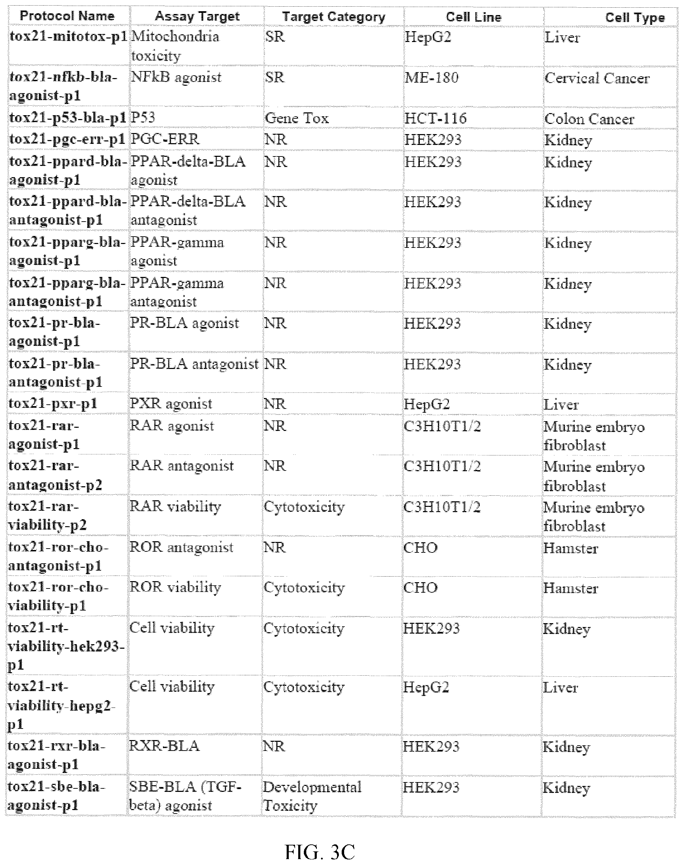

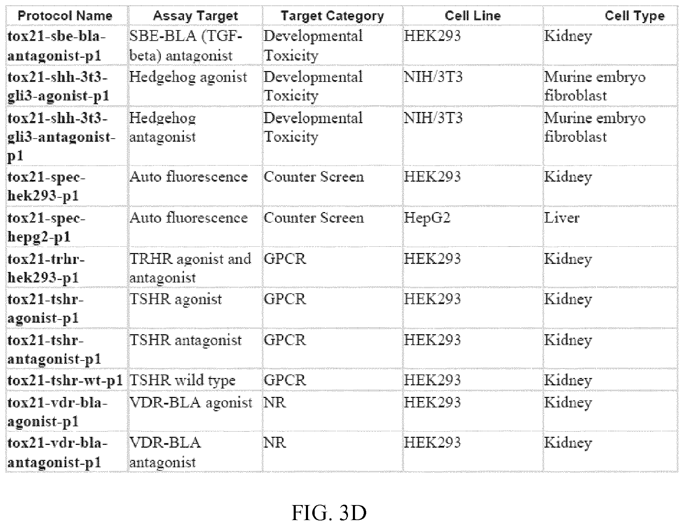

In some embodiments, the clinical trial success/failure model can be considered as a set of models that are its inputs, as shown in the table in Figures 3A-3D. The system or method (e.g., application) can provide summaries for a comprehensive set of toxicological endpoints in addition to the output from the main, clinical success/failure model.

In some embodiments, the system or method can be considered for specialized cases where the user, for example, selects the option that they are evaluating ingredients for personal care products using the toxicological endpoints described above and shown in the table in Figures 3A-3D to identify safer alternatives.

In an embodiment, the system or method can be used to infer biochemical pathways (rather than proteins). That is, the complex output can be summarized into simplified toxicological categories and broader systems possibly affected by the candidate chemical(s) and/or drug(s). This makes the system or method more accessible and useful to non-specialists and/or non-toxicologists.

In an embodiment, the system and method can bypass or omit any web interface, offering any or all of the aforementioned tools to the user through an application programming interface (API). The API can require a user-specific token for security but may otherwise be called in various external software environments (e.g., GoogleSheets), populating spreadsheets with the outputs herein described.

In certain embodiments, the user can upload their own toxicological data to build custom machine learning models (e.g., for biological targets not listed in the table shown in Figures 3A-3D).

In an embodiment, a system or method can include a user interface (e.g., a cloud-based software interface) and can utilize one or more models (e.g., machine learning models) that predict potential clinical trial failures for at least one candidate chemical and/or drug. A user can load activity data from any arbitrary toxicological tests and train at least one model of the one or more models. A model predicting clinical trial success can, for example, use estimates from one or more human protein targets in the table in Figures 3A-3D: AhR, AP-1 agonist, AR-BLA agonist, AR-BLA antagonist, AR-MDA agonist, AR-MDA agonist (with antagonist), AR-MDA antagonist, AR-MDA antagonist (lower agonist), ARE, Aromatase, CAR agonist, CAR antagonist, Caspase-3/7, Cell viability, ATAD5, ER-BLA agonist, ER-BLA antagonist, ER-BG1 agonist, ER-BG1 agonist (with antagonist), ER-BG1 antagonist, ER-BG1 antagonist (lower agonist), ER-beta antagonist, ER-beta agonist, ERR, ER stress, FXR-BLA agonist, FXR-BLA antagonist, TR-beta agonist, TR-beta antagonist, GR-BLA agonist, GR-BLA antagonist, H2AX, HDAC, HRE-BLA agonist, HSE-BLA, Luciferase, biochemical, Mitochondria toxicity, NFkB agonist, P53, PGC-ERR, PPAR-delta-BLA agonist, PPAR-delta-BLA antagonist, PPAR-gamma agonist, PPAR-gamma antagonist, PR-BLA agonist, PR-BLA antagonist, PXR agonist, RAR agonist, RAR antagonist, RAR viability, ROR antagonist, ROR viability, Cell viability, Cell viability, RXR-BLA, SBE-BLA (TGF-beta) agonist, SBE-BLA (TGF-beta) antagonist, Hedgehog agonist, Hedgehog antagonist, TRHR agonist and antagonist, TSHR agonist, TSHR antagonist, TSHR wild type, VDR-BLA agonist, and/or VDR-BLA antagonist. Chemicals and/or drugs can be uploaded by the user. The one or more models (e.g., the protocols listed in the table in Figures 3A-3D) can be used to generate summary reports that include broad toxicological risk categories. The summary reports can include pathway analysis, estimating for the use the biochemical pathways that may be affected by the uploaded chemical(s) and/or drug(s). The summary reports can include, for example, estimated vapor pressure and estimates for blood brain barrier permeability, dermal absorption and irritation, cardiac and kidney toxicity, rat LD50, a measure of lethality, liver toxicity, and eye irritation.

Embodiments of the subject invention provide a focused technical solution to the focused technical problem of how to increase availability and accessibility of clinical trial outcomes on chemicals (which can include drugs) that may be present in supplements and/or personal care products without increasing costs.

The methods and processes described herein can be embodied as code and/or data. The software code and data described herein can be stored on one or more machine-readable media (e.g., computer-readable media), which may include any device or medium that can store code and/or data for use by a computer system. When a computer system and/or processor reads and executes the code and/or data stored on a computer-readable medium, the computer system and/or processor performs the methods and processes embodied as data structures and code stored within the computer-readable storage medium.

It should be appreciated by those skilled in the art that computer-readable media include removable and non-removable structures/devices that can be used for storage of information, such as computer-readable instructions, data structures, program modules, and other data used by a computing system/environment. A computer-readable medium includes, but is not limited to, volatile memory such as random access memories (RAM, DRAM, SRAM); and nonvolatile memory such as flash memory, various read-only-memories (ROM, PROM, EPROM, EEPROM), magnetic and ferromagnetic/ferroelectric memories (MRAM, FeRAM), and magnetic and optical storage devices (hard drives, magnetic tape, CDs, DVDs); network devices; or other media now known or later developed that are capable of storing computer-readable information/data. Computer-readable media should not be construed or interpreted to include any propagating signals. A computer-readable medium of embodiments of the subject invention can be, for example, a compact disc (CD), digital video disc (DVD), flash memory device, volatile memory, or a hard disk drive (HDD), such as an external HDD or the HDD of a computing device, though embodiments are not limited thereto. A computing device can be, for example, a laptop computer, desktop computer, server, cell phone, or tablet, though embodiments are not limited thereto.

When ranges are used herein, such as for dose ranges, combinations and subcombinations of ranges (e.g., subranges within the disclosed range), specific embodiments therein are intended to be explicitly included. When the term "about" is used herein, in conjunction with a numerical value, it is understood that the value can be in a range of 95% of the value to 105% of the value, i.e. the value can be +/- 5% of the stated value. For example, "about 1 kg" means from 0.95 kg to 1.05 kg.

It should be understood that the examples and embodiments described herein are for illustrative purposes only and that various modifications or changes in light thereof will be suggested to persons skilled in the art and are to be included within the spirit and purview of this application.

All patents, patent applications, provisional applications, and publications referred to or cited herein are incorporated by reference in their entirety, including all figures and tables, to the extent they are not inconsistent with the explicit teachings of this specification.

## Claims

1. A system for predicting clinical trial outcomes for chemicals, the system comprising:
a processor; and
a machine-readable medium in operable communication with the processor and having instructions stored thereon that, when executed by the processor, perform the following steps:
utilizing at least one machine learning model on known toxicological test results to develop a prediction model;
receiving data of a candidate chemical; and
utilizing the prediction model on the candidate chemical to predict a clinical trial outcome for the candidate chemical.

2. The system according to claim 1, wherein the known toxicological test results are uploaded to the system by a user of the system.

3. The system according to any of claims 1-2, wherein the known toxicological test results are test results on human protein targets.

4. The system according to any of claims 1-3, further comprising a display in operable communication with the processor,
wherein the instructions when executed further perform the step of displaying the predicted clinical trial outcome on the display.

5. The system according to any of claims 1-4, wherein the instructions when executed further perform the step of generating summary reports, and
wherein the summary reports comprise broad toxicological risk categories.

6. The system according to claim 5, wherein the summary reports comprise pathway analysis and estimating for use biochemical pathways that are affected by the candidate chemical.

7. The system according to any of claims 5-6, wherein the summary reports comprise at least one of estimated vapor pressure, blood brain barrier permeability, and toxicological estimates.

8. The system according to any of claims 5-6, wherein the summary reports comprise additional whole organ or *in vivo* toxicological estimates including at least one of dermal absorption and irritation, cardiac and kidney toxicity, mammalian LD50, a measure of lethality, liver toxicity, and eye irritation.

9. The system according to any of claims 1-8, wherein the candidate chemical is a drug.

10. A method for predicting clinical trial outcomes for chemicals, the method comprising:
utilizing at least one machine learning model on known toxicological test results to develop a prediction model;
receiving data of a candidate chemical; and
utilizing the prediction model on the candidate chemical to predict a clinical trial outcome for the candidate chemical.

11. The method according to claim 10, wherein the known toxicological test results are uploaded to the system by a user.

12. The method according to any of claims 10-11, wherein the known toxicological test results are test results on human protein targets.

13. The method according to any of claims 10-12, further comprising displaying the predicted clinical trial outcome on a display.

14. The method according to any of claims 10-13, further comprising generating summary reports,
wherein the summary reports comprise:
broad toxicological risk categories;
pathway analysis and estimating for use biochemical pathways that are affected by the candidate chemical;
at least one of estimated vapor pressure, blood brain barrier permeability, and toxicological estimates; and/or
additional whole organ or *in vivo* toxicological estimates including at least one of dermal absorption and irritation, cardiac and kidney toxicity, mammalian LD50, a measure of lethality, liver toxicity, and eye irritation.

15. The method according to any of claims 10-14, wherein the candidate chemical is a drug.
